(19) **Europäisches Patentamt**
**European Patent Office**
**Office européen des brevets**

(11) **EP 1 316 547 A1**

(12) **EUROPEAN PATENT APPLICATION**
published in accordance with Art. 158(3) EPC


(43) Date of publication:
**04.06.2003 Bulletin 2003/23**

(21) Application number: **01963509.3**

(22) Date of filing: **07.09.2001**

(51) Int Cl.[7]: **C07C 259/06**

(86) International application number:
**PCT/JP01/07775**

(87) International publication number:
**WO 02/020467 (14.03.2002 Gazette 2002/11)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **08.09.2000 JP 2000273445**



(71) Applicant: **ONO PHARMACEUTICAL CO., LTD.**
**Osaka-shi, Osaka 541-8526 (JP)**

(72) Inventors:
- **OKUYAMA,S Fukui Res.Ins.**
  **Ono Pharmaceutical Co.Ltd**
  **Sakai-gun, Fukui 913-003 (JP)**
- **OKAMOTO,M Fukui Res.Ins.**
  **Ono Pharmaceutical Co.Ltd**
  **Sakai-gun, Fukui 913-003 (JP)**

(74) Representative: **Henkel, Feiler, Hänzel**
**Möhlstrasse 37**
**81675 München (DE)**


(54) **NOVEL CRYSTALS OF N-HYDROXY-2(S)-METHYL-5-ETHOXYMETHOXY -4(S)-N-(4-PHENOXYPHENYLCARBONYL)AMINO]PENTANAMIDE, PROCESS FOR THEIR PRODUCTION AND MEDICINES CONTAINING THE CRYSTALS AS THE ACTIVE INGREDIENT**


(57) The present invention relates to a novel crystal of a compound of formula (I).

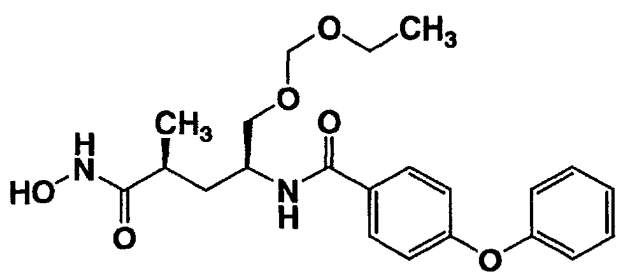

(I)

The present invention can provide a compound of formula (I), which is useful as a pharmaceutical (matrix metalloproteinase inhibitor) as a stable crystal which possesses a constantly the same quality.


FIG. 11

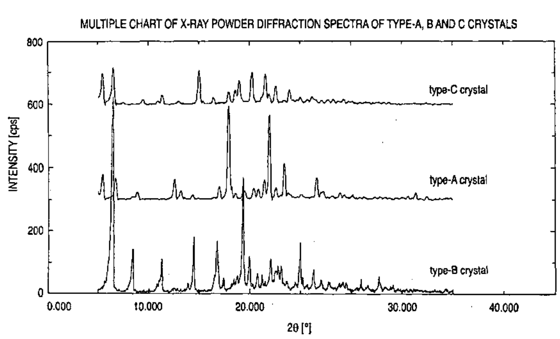


EP 1 316 547 A1

## Description

Technical Field

The present invention relates to a novel crystal of N-hydroxy-2(S)-methyl-5-ethoxymethoxy-4(S)-[N-(4-phenoxyphenylcarbonyl)amino]pentanamide.

More specifically, the present invention relates to

(1) novel crystals, i.e. a type-A crystal and a type-B crystal of N-hydroxy-2(S)-methyl-5-ethoxymethoxy-4(S)-[N-(4-phenoxyphenylcarbonyl)amino]pentanamide of formula (I) (referred to as compound (I) hereafter),
(2) a method for the preparation thereof and
(3) a pharmaceutical agent comprising the same as active ingredient.

Background

The compound (I) is a promising compound as a pharmaceutical, for example, WO 99/19296 discloses that the compound (I) has an inhibitory activity against matrix metalloproteinase (abbreviated as MMP hereafter) and it is useful for the treatment and/or prophylaxis of an agent for the treatment and/or prophylaxis of rheumatoid arthritis, arthrosteitis, pathological bone resorption, osteoporosis, periodontitis, interstitial nephritis, arteriosclerosis, pulmonary emphysema, cirrhosis hepatis, corneal damages, diseases of metastasis and infiltration of cancer cells and proliferation, autoimmune diseases (Crohn's diseases, Sjogren's syndrome, etc.), diseases caused by vascular emigration or infiltration of leukocytes, angiogenesis, multiple sclerosis, aortic aneurysm and endometriosis.

In the specification of PCT/JP01/00914, it is described that the compound (I) is useful as an agent for the treatment and/or prophylaxis of osteoarthritis.

In the specification of PCT/JP01/02946, the compound (I) is useful for the treatment and/or prophylaxis of those diseases caused by constrictive vessel lesion, e.g. restenosis after PTCA, unstable angina, acute myocardial infarction and transient ischemic attack.

However, in the above literature no description or implication is found whether the compound (I) has some polymorphs or not.

Generally, polymorphs of a compound may show different physical properties from each other. Particularly, in pharmaceutical fields, it is known that action intensity may vary, e.g. solubility, rate of dissolving, stability, absorbability, etc. Therefore, even when the same compound is used, there may be cases wherein action intensity desired is not given or different action intensity from prediction is caused, resulting in unexpected situation because of the varieties of polymorphs. Therefore it is desired to provide with a compound having uniformed quality which is expected to have constant action intensity.

Therefore, when a compound which possesses some polymorphs, in order to secure uniformity of quality and a uniformed action intensity which are required as pharmaceuticals, it is necessary to provide with a compound which has uniformed crystal form constantly. From a point of view of preservation, it is desired to provide a crystal which has uniformed quality.

The present inventors have investigated on the compound (I) to find out that the compound (I) has several polymorphs, i.e. a type-A crystal, a type-B crystal and a type-C crystal.

On the other hand, example 71 in the specification of WO 99/19296 discloses the method for the preparation of compound (I) specifically, but nothing is described about the existence of polymorphs. The present inventors synthesized compound (I) according to the method in example 71 of the above specification and investigated the crystal form of compound (I), and then it proved that it was the type-C crystal.

The present inventors have investigated on each crystals of type-A, B and C, so that the present inventors have found that each crystal of type-A, B and C were prepared selectively by recrystallization, and that in order to prepare a uniformed crystal, a seed crystal is required for the type-C crystal, but no seed crystal is required for the type-A and B crystals.

If a compound has electrostatic property, then for example, it may cause a problem such as sticking of the compound to a stirring bar when subjected to stirring in the manufacture or adhesion and aggregation in formulation, so in the manufacture in industrial scale those compounds which have weak electrostatic property are desired. And it was confirmed that the type-A crystal was excellent when stability and oral absorbability are tested.

From these above, it was found that in order to provide compound (I) in a constant uniformed quality as a stable pharmaceutical bulk, the type-A and type-B crystals are preferable and the type-A crystal is excellent.

The present inventors have found the method for the preparation of uniformed type-A crystal and type-B crystal.

Disclosure of the invention

The present invention related to

(1) a novel type-A and type-B crystal of N-hydroxy-2(S)-methyl-5-ethoxymethoxy-4(S)-[N-(4-phenoxyphenylcarbonyl)amino]pentanamide of formula (I)

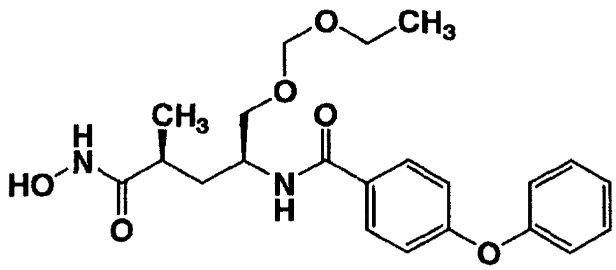

(I)

(referred to as compound (I) hereafter),
(2) a method for the preparation thereof and
(3) a pharmaceutical agent comprising the same as active ingredient.

The type-A crystal of N-hydroxy-2(S)-methyl-5-ethoxymethoxy-4(S)-[N-(4-phenoxyphenylcarbonyl)amino] pentanamide is characterized by the data of diffraction angle (2 θ ), half bandwidth and relative intensity, shown in the following table 1.

Table 1

| diffraction angle (2θ) | half bandwidth | relative intensity |
|---|---|---|
| 5.4 | 0.3 | medium |
| 6.7 | 0.2 | medium |
| 12.5 | 0.3 | medium |
| 17.0 | 0.3 | medium |
| 17.9 | 0.3 | strong |
| 21.4 | 0.3 | medium |
| 21.9 | 0.3 | strong |
| 23.4 | 0.3 | rather strong |
| 26.6 | 0.3 | medium |

The type-B crystal of N-hydroxy-2(S)-methyl-5-ethoxymethoxy-4(S)-[N-(4-phenoxyphenylcarbonyl)amino] pentanamide is characterized by the data of diffraction angle (2θ) using Cu-Kα ray, half bandwidth and relative intensity, shown in the following table 2.

Table 2

| diffraction angle (2θ) | half bandwidth | relative intensity |
|---|---|---|
| 6.5 | 0.1 | strong |
| 8.4 | 0.1 | medium |
| 11.3 | 0.1 | medium |
| 14.5 | 0.1 | medium |
| 16.8 | 0.2 | medium |
| 19.3 | 0.1 | rather strong |

Table 2 (continued)

| diffraction angle (2θ) | half bandwidth | relative intensity |
|---|---|---|
| 20.2 | 0.1 | medium |
| 22.1 | 0.2 | medium |
| 25.0 | 0.1 | medium |

The type-A and type-B crystals of N-hydroxy-2(S)-methyl-5-ethoxymethoxy-4(S)-[N-(4-phenoxyphenylcarbonyl)amino]pentanamide are characterized by physiochemical properties described in the present specification, but the spectra data may vary judging from the nature of the spectra.

For example, judging from the nature of X-ray powder diffraction spectrum, it is important for the recognition of identity of the crystals to read the diffraction angle (2 θ ), half bandwidth and overall patterns and the relative intensity may vary to some degree subjecting to the direction of crystal growth, particle size, and conditions on measurement.

In data of differential scanning calorimetry (DSC), overall pattern is important for the recognition of crystal identity and it may depend on the condition of measurement.

Furthermore, in infrared absorption (IR) spectrum, overall pattern is important for the recognition of identifying crystals and therefore it may vary to some degree subjecting to the condition of measurement.

Therefore, those crystals which have analogous data and patterns in X-ray diffraction spectrum, differential scanning calorimetry (DSC) or infrared absorption (IR) spectrum with those of the type-A crystal and the type-B crystal, are included in the type-A crystal and the type-B crystal of the present invention.

Brief description of the figures

Fig.1 shows X-ray powder diffraction spectrum of the type-A crystal.
Fig.2 shows differentiation scanning calorimetry (DSC) chart of the type-A crystal.
Fig.3 shows infrared absorption (IR) spectrum of the type-A crystal.
Fig.4 shows X-ray powder diffraction spectrum of the type-B crystal.
Fig.5 shows differentiation scanning calorimetry (DSC) chart of the type-B crystal.
Fig.6 shows infrared absorption (IR) spectrum of the type-B crystal.
Fig.7 shows X-ray powder diffraction spectrum of the type-C crystal.
Fig.8 shows differential scanning calorimetry (DSC) chart of the type-C crystal.
Fig.9 shows infrared absorption (IR) spectrum of the type-C crystal.
Fig.10 shows multiple chart of infrared absorption (IR) spectrum of the crystals prepared in reference example 2 and comparative example 1.
Fig.11 shows multiple chart of X-ray powder diffraction spectrum of the type-A, B and C crystals.
Fig.12 shows multiple chart of differential scanning calorimetry (DSC) of the type-A, B and C crystals.
Fig.13 shows multiple chart of infrared absorption ($I_R$) spectrum of the type-A, B and C crystals.
Fig.14 shows endothermic peak chart by differential scanning calorimetry (DSC) of the type-A crystal.
Fig.15 shows endothermic amount chart by differential scanning calorimetry (DSC) of the type-A crystal, which was preserved under the condition of 40 °C-75%RH, for 1 month.
Fig.16 shows endothermic amount chart by differential scanning calorimetry (DSC) of the type-A crystal, which was preserved under the condition of 40 °C, for 6 months.
Fig.17 (A) shows X-ray powder diffraction spectrum of the type-A crystal and (B) shows X-ray powder diffraction spectrum of the type-A crystal, which was preserved at 40 °C for 6 months.
Fig.18 shows the graph which represents the transition of concentrations of the type-A and type-C crystals in plasma.

Description of the present invention

Each crystal of the type-A, B or C of the compound (I) is prepared by the method described in examples or the following method.

That is, the type-A crystal, the type-B crystal and the type-C crystal may be prepared by recrystallization of compound (I) in a mixed solvent of rich solvent, which solvates crude compound (I), and poor solvent, which does not solvate it.

Concretely, the type-A crystal may be prepared by recrystallizing crude compound (I) in a mixed solvent of alcohol solvent (methanol, ethanol, propanol, isopropanol, butanol, t-butanol, etc.) and water, or in a mixed solvent of tetrahydrofuran and cyclohexane.
Preferable alcohol solvent includes ethanol and isopropanol.
Preferable ratio of mixed solvent in volume is, alcohol solvent : water = 1 : 3 ~ 3 : 1, and more preferably 1 : 2.
Preferable ratio of mixed solvent in volume is, tetrahydrofuran : cyclohexane = 2 : 1.
Preferable solvent volume of a mixture of alcohol solvent and water and a mixture of tetrahydrofuran and cyclohexane is, approximately 2 ～ 50 ml versus 1 g of crude compound (I), and more preferably approximately 30 ml.
The type-B crystal may be prepared by recrystallizing crude compound (I) in a mixed solvent of tetrahydrofuran and cyclohexane.
Preferable ratio of a mixed solvent in volume is tetrahydrofuran : cyclohexane = 3 : 2.
The volume of a mixed solvent is approximately 2～100 ml per 1 g of compound (I), more preferably approximately 4～48 ml, more preferably approximately 10 ml.

[Pharmaceutical effect]

It was confirmed that the compound of the present invention has matrix metalloproteinase inhibitory activity by the method described in pages 149~151 of the specification of WO 99/19296.

[Toxicity]

The compound of the present invention is very low and therefore it is safe enough for pharmaceutical use.

[Pharmaceutical Use]

The compound of the present invention has inhibitory activity against matrix metalloproteinase and therefore it is expected to be used as an agent for the treatment and/or prophylaxis of rheumatoid arthritis, arthrosteitis, pathological bone resorption, osteoporosis, periodontitis, interstitial nephritis, arteriosclerosis, pulmonary emphysema, cirrhosis hepatis, corneal damages, diseases of metastasis and infiltration of cancer cells and proliferation, autoimmune diseases (Crohn's diseases, Sjogren's syndrome, etc.), diseases caused by vascular emigration or infiltration of leukocytes, angiogenesis, multiple sclerosis, aortic aneurysm, endometriosis, constrictive vessel lesion, e.g. restenosis after PTCA, unstable angina, acute myocardial infarction, transient ischemic attack.
For the purpose described above, the compounds of the present invention, may normally be administered systemically or topically, usually by oral or parenteral administration.
The doses to be administered are determined depending upon, for example, age, body weight, symptom, the desired therapeutic effect, the route of administration, and the duration of the treatment. In the human adult, the doses per person are generally from 1 mg to 1000 mg, by oral administration, up to several times per day, and from 1 mg to 100 mg, by parenteral administration (preferably intravenous administration), up to several times per day, or continuous administration for from 1 to 24 hours per day from vein.
As mentioned above, the doses to be used depend upon various conditions. Therefore, there are cases wherein doses lower than or greater than the ranges specified above may be used.
The compound of the present invention may be administered in the form of solid compositions for oral administration or parenteral administration.
Solid compositions for oral administration include compressed tablets, pills, capsules, dispersible powders and granules, etc.
Capsules include hard capsules and soft capsules.
In such solid compositions, one or more of the active compound(s) may be admixed with at least one inert diluent (e.g. lactose, mannitol, glucose, hydroxypropyl cellulose, microcrystalline cellulose, starch, polyvinyl pyrrolidone or magnesium metasilicate aluminate). The compositions may also comprise, as is normal practice, additional substances other than inert diluents: e.g. lubricating agents (e.g. magnesium stearate), disintegrating agents (e.g. cellulose calcium glycolate), stabilizing agents (e.g. lactose), and agents to assist dissolution (e.g. glutamic acid or asparatic acid). The tablets or pills may, if desired, be coated with a film of gastric or enteric material (e.g. sugar, gelatin, hydroxypropyl cellulose or hydroxypropylmethyl cellulose phthalate), or be coated with two or more films. Further, coating may include containment within capsules of absorbable materials such as gelatin.
Solid compositions for parenteral administration include suppositories for rectal administration and pessaries for vaginal administration which comprise one or more of the active compound(s) and may be prepared by methods known *per se.*

Best mode for carrying out the invention

The following reference examples, examples, comparative examples and test examples illustrate the new crystal of compound (I) of the present invention, but the present invention are not limited to them.

Reference example 1 : Preparation example of N-hydroxy-2(S)-methyl-5-ethoxymethoxy-4(S) -[N-(4-phenoxyphenylcarbonyl)amino]pentanamide

In a 100 L reaction pot was added N-(1-methoxy-1,1-dimethylmethyl)oxy-2(S)-methyl-5-ethoxymethoxy-4(S) -[N-(4-phenoxyphenylcarbonyl)amino]pentanamide (5100 g; prepared according to the method described in the specification of WO 99/19296) and methanol (15 L) and the mixture was dissolved by stirring.
To the above solution was added 1M aqueous hydrochloric acid (313 ml) slowly and the mixture was stirred for 1 hour at room temperature. After confirming the termination of the reaction, the reaction mixture was filtrated.
The filtrate was washed with methanol (5 L). The filtrate and washed solution was combined and it was added to a 100 L reaction pot. Hereto was added tap water (3 L) and hexane (10 L). The above solution was stirred and was allowed to stand and was separated. The methanol layer was washed with hexane (10 L). The methanol layer was poured into tap water (80 L) under stirring slowly and the mixture was stirred for 30 minutes with internal temperature 20-30 °C and the mixture was cooled down to 0-5 °C and the mixture was stirred for 30 minutes. The mixture was centrifuged to give a crystal and was washed with tap water (20 L).
The crystal was dried for 15 hours to give a crude crystal of N-hydroxy-2(S)-methyl-5-ethoxymethoxy-4(S) -[N-(4-phenoxyphenylcarbonyl)amino]pentanamide (4044 g).

Example 1 : Preparation of type-A crystal

In a 300 L dissolution pot was added isopropanol (27 L) and tap water (27 L) (50% aqueous solution of isopropanol) and thereto was added crude crystal of N-hydroxy-2(S)-methyl-5-ethoxymethoxy-4(S)-N-(4-phenoxyphenylcarbonyl)amino]pentanamide (3.6 kg), which was prepared in reference example 1 and the mixture was dissolved by stirring with heating to adjust internal temperature 50 °C. After the substance was completely dissolved, the mixture was cooled down to adjust internal temperature 40 °C. To another 300 L pot for crystallization were added isopropanol (11 L) and tap water (43 L) (aqueous 20% solution of isopropanol) and the internal temperature was adjusted to 20~25 °C. The solution in the pot was added to the above pot for crystallization with the internal temperature 20~35 °C through 50L filter press which was warmed to approximately 40 °C. The dissolution pot and filter press were washed with 50% aqueous solution of isopropanol and the solution was added to the pot for crystallization. The internal temperature of crystallization pot was lowered to 20 ~ 25 °C and the mixture was stirred for 1 hour. After confirming that the crystal precipitated to a great degree, the mixture was cooled down to 0~5 °C (internal temperature) and the mixture was stirred for 30 minutes. The precipitated crystal was centrifuged. The crystal which separated out was washed with 35% aqueous solution of isopropanol (7.2 L). The crystal was dried under reduced pressure for more than 15 hours at 40 °C to give type-A, crystal of N-hydroxy-2(S)-methyl-5-ethoxymethoxy-4(S)-[N-(4-phenoxyphenylcarbonyl)amino]pentanamide (3.01 kg).

Physical data of type-A crystal

1) melting point : 137~140 °C
2) X-ray powder diffraction spectrum measured by the following condition is shown in fig.1, the chart of differential scanning calorimeter (DSC) in fig.2 and infrared absorption (IR) spectrum is shown in fig.3, each was measured under the following condition.

(1) X-ray powder diffraction spectrum

Apparatus : Rigaku X-ray powder diffraction apparatus RAD-2C,
Target: Cu,
Filter: not used,
Voltage: 40 kV,
electrical current : 20 mA,
scanning speed : 2.0° /min

The chart of fig.1 is given after smoothing and removal of background.

(2) Examining of Differential Scanning Calorimetry (DSC)

Apparatus : Shimadzu DSC-50,
Sample: 3.90 mg,
Sample cell : aluminum open cell,
Nitrogen Gas Flow : 20 ml/min,
heating rate : 10 °C/min.

(3) Infrared absorption (IR) spectrum

Apparatus : ASI APPLIED SYSMTEMS REACT IR1000(brand name)
Dissolution performance : 2 $cm^{-1}$
Scanning number of times : 128

Example 2 : Preparation of B-type crystal

To tetrahydrofuran (18 ml) was added crude crystal of N-hydroxy-2(S)-methyl-5-ethoxymethoxy-4(S)-[N-(4-phenoxyphenylcarbonyl)amino]pentanamide (2.86 g) and the mixture was dissolved by heating. To the mixture was added cyclohexane (12 ml) slowly with internal temperature 62 °C and was cooled down slowly. A crystal started to precipitate (separate out) with internal temperature 53 °C. When the internal temperature was below approximately 40 °C, to the mixture was added a mixed solvent (tetrahydrofuran : cyclohexane = 1 : 1, 10 ml). With internal temperature 25 °C the mixture was stirred for 30 minutes and the given crystal was collected off under reduced pressure. Thus obtained crystal was washed with a mixed solvent (tetrahydrofuran : cyclohexane =1 : 1, 10 ml) and was dried under reduced pressure at room temperature to give type-B crystal of N-hydroxy-2(S)-methyl-5-ethoxymethoxy-4(S)-[N-(4-phenoxyphenylcarbonyl)amino]pentanamide (2.38 g).

Physical data of type-B crystal

1) melting point : 141 ~ 143 °C
2) Fig.4 shows the X-ray powder diffraction spectrum, fig.5 shows the chart of differential scanning calorimetry (DSC) and fig.6 shows the infrared absorption (IR) spectrum, all of which were measured under the following conditions, respectively.

(1) X-ray powder diffraction spectrum

Apparatus : Rigaku X-ray powder diffraction apparatus RAD-2C,
Target : Cu,
Filter : not used,
Voltage : 40 kV,
Electric current : 20 mA,
Scanning speed : 2.0 °C/min

The chart of fig.4 is given after smoothing and removal of background.

(2) Differential Scanning Calorimetry (DSC)

Apparatus : Shimadzu DSC-50,
Sample : 4.54 mg,
Sample cell : aluminum open cell,
Nitrogen gas flow: 20 ml/min,
Heating rate : 10 °C/min.

(3) Infrared absorption (IR) spectrum

Apparatus : ASI APPLIED SYSTEMS REACT IR1000 (brand name)
Dissolution performance : $2cm^{-1}$
Scanning number of times : 128

Reference example 2 : Preparation of type-C crystal

To ethyl acetate (40 ml) was added crude crystal of N-hydroxy-2(S)-methyl-5-ethoxymethoxy-4(S)-[N-(4-phenoxyphenylcarbonyl)amino]pentanamide (2.0 g), which was prepared in reference example 1, and the mixture was dissolved by heating. This solution was cooled down slowly and thereto was added seed crystal of type C crystal (20 mg) with internal temperature 65 °C. This solution was cooled down slowly to give crystallization. The solution was cooled down to 0~5 °C and the mixture was stirred for 30 minutes with internal temperature 5 °C and the crystal was collected off. The given crystal was washed with cool ethyl acetate (4.0 ml). The crystal was dried under reduced pressure at room temperature to give type-C crystal of N-hydroxy-2(S)-methyl-5-ethoxymethoxy-4(S)-[N-(4-phenoxyphenylcarbonyl)amino]pentanamide (1.54 g).

Physical data of type-C crystal

1) melting point : 138 ~ 142 °C
2) Fig.7 shows the X-ray powder diffraction spectrum, fig.8 shows the chart of differential scanning calorimetry (DSC) and fig.9 shows infrared absorption (IR) spectrum, all of which were measured under the following conditions, respectively.

(1) X-ray powder diffraction spectrum

Apparatus : Rigaku X-ray powder diffraction apparatus RAD-2C,
Target : Cu,
Filter : not used,
voltage : 40 kV,
Electric current : 20 mA,
Scanning speed : 2.0 °C/min

The chart of fig.7 is given after smoothing and removal of background.

(2) Differential Scanning Calorimetry (DSC)

Apparatus : Shimadzu DSC-50,
Sample : 3.49 mg,
Sample cell : aluminum open cell,
Nitrogen gas flow: 20 ml/min,
Heating rate : 10 °C/min.

(3) Infrared absorption (IR) spectrum

Apparatus : ASI APPLIED SYSTEMS REACT IR1000 (brand name)
Dissolution performance : $2cm^{-1}$
Scanning number of times : 128

Comparative example 1 :

As shown below, N-hydroxy-2(S)-methyl-5-ethoxymethoxy-4(S)-[N-(4-phenoxyphenylcarbonyl)amino]pentanamide was synthesized according to the method described in example 71 of the specification of WO 99/19296.
To a solution of N-(1-methoxy-1,1-dimethylmethyl)oxy-2(S)-methyl-5-ethoxymethoxy-4(S)-[N-(4-phenoxyphenylcarbonyl)amino]pentanamide (1.012 g) in methanol (40 ml) was added 1N hydrochloric acid (8 drops). The reaction mixture was stirred at room temperature. After confirming that the starting material disappeared, the reaction mixture was concentrated. The given residue was washed with diethyl ether (30 ml) to give N-hydroxy-2(S)-methyl-5-ethoxymethoxy-4(S)-[N-(4-phenoxyphenylcarbonyl)amino]pentanamide (709 mg).

As a result of measurement of infrared absorption spectrum on N-hydroxy-2(S)-methyl-5-ethoxymethoxy-4 (S)-[N-(4-phenoxyphenylcarbonyl)amino]pentanamide, it proved that the spectrum was the same as that of type-C crystal, which was given in reference example 2 (see fig.10).
Table 3 shows the summary of data regarding crystal forms of type-A, B and C crystals.
And fig.11 shows multiple chart of X-ray powder diffraction spectra on type-A, B and C, fig.12 shows multiple chart of differential scanning calorimetry (DSC) and fig.13 shows multiple chart of infrared absorption (IR) spectra.

Table 3

| | type-A crystal | | | type-B crystal | | | type-C crystal | | |
|---|---|---|---|---|---|---|---|---|---|
| X-ray powder diffraction spectrum | see fig.1 | | | see fig.4 | | | see fig.7 | | |
| | diffraction angle ($2\theta$) | half band-width | relative intensity | diffraction angle ($2\theta$) | half band-width | relative intensity | diffraction angle ($2\theta$) | half band-width | relative intensity |
| | 5.4 | 0.3 | 27 | 6.5 | 0.1 | 100 | 5.4 | 0.3 | 87 |
| | 6.7 | 0.2 | 23 | 8.4 | 0.1 | 24 | 6.5 | 0.3 | 100 |
| | 12.5 | 0.3 | 22 | 11.3 | 0.1 | 19 | 11.3 | 0.3 | 28 |
| | 17.0 | 0.3 | 14 | 14.5 | 0.1 | 31 | 14.9 | 0.2 | 93 |
| | 17.9 | 0.3 | 100 | 16.8 | 0.2 | 29 | 17.9 | 0.3 | 37 |
| | 21.4 | 0.3 | 21 | 19.3 | 0.1 | 63 | 18.5 | 0.3 | 41 |
| | 21.9 | 0.3 | 90 | 20.0 | 0.1 | 20 | 18.9 | 0.3 | 67 |
| | 23.4 | 0.3 | 39 | 22.1 | 0.2 | 19 | 20.2 | 0.3 | 89 |
| | 26.6 | 0.3 | 24 | 25.0 | 0.1 | 28 | 21.5 | 0.3 | 84 |
| | | | | | | | 22.6 | 0.3 | 52 |
| | | | | | | | 23.9 | 0.2 | 42 |
| melting point (°C) | 137-140 | | | 141-143 | | | 138-142 | | |
| DSC endothermic peak temperature (°C) | see fig.2<br>130.1<br>141.4<br>149.0 | | | see fig.5<br>150.1 | | | see fig.8<br>131.3<br>142.4<br>148.5 | | |
| infrared absorption spectrum | see fig.3 | | | see fig.6 | | | see fig.9 | | |

As is apparent from table 3, fig. 1~9 and fig.11~13, type-A, B and C crystals have different crystal forms from each other.

Stability test :

Weighing out over approximately 50 mg of type-A crystal powder, it was preserved under the condition of

1) 40 °C, 75%RH (relative humidity), unsealed for a month,
2) 40 °C, sealed tightly for 6 months.

The type-A crystal preserved under above conditions and another type-A crystal which was preserved at 5 °C were subjected to measurement of differential scanning calorimetry (DSC) and caloric transition (ΔH) per 1 g was calculated from endothermic peak chart (see fig.14~16)

And fig.17(A) shows X-ray diffraction spectrum of type-A crystal and (B) shows X-ray diffraction spectrum of type-A crystal, which was preserved for 6 months at 40 °C.

$$\Delta H = \text{peak area} / \text{sample weight}$$

Table 4 shows the calorie variation (absolute value) of peaks at each condition.

Table 4

| | ΔH (J/g) |
|---|---|
| initial | 13.40 |
| 1 month, 40°C-75%RH | 14.80 |
| 6 months, 40°C | 13.41 |

As is apparent from DSC chart, type-A crystal is transited to type-C crystal and type-B crystal, and therefore it is generally thought to be unstable. However, as is seen from above table 4, no significant difference in calorie variety is recognized neither in 40 °C-75%RH-a month nor in 40°C-6 month preservation (see fig.17) and so crystal transition is not caused so it proved to be a stable crystal.

Oral absorption test :

The compound of the present invention (2 g), DK ester SS (Daiichi Kogyo Seiyaku) and lactose (2.9 g) were weighed and the mixture was mixed slightly in a mortar and the mixture was filtered through 60 mesh strainer twice to give pharmaceutical powder. The pharmaceutical powder was filled in No.0 gelatin capsule 250 mg/cap each. By this method, capsule formulations containing type-A and type-C crystal were prepared respectively.

A beagle was fed with solid food (240 g) and after 30 minutes above prepared capsules were administered in a stomach using sonde for oral administration and tap water (20 ml) was administered with sonde for oral administration.

After administration, 15 minutes, 30 minutes, 1 hour, 2 hours and 3 hours taken a blood of 1 ml from cephalic vein using heparin dealt syringe. The taken sample was disposed under 12000 rpm for 2 minutes and plasma was separated and the concentration of the compound in plasma (μg/ml) was measured.

The dose given to the beagle was 50 mg/kg and 4 beagles for type-A crystal and 3 beagles for type-C crystal. The transition of blood-concentration-time was shown in fig.18.

And table 5 shows the average value of area under the (blood concentration-time) curve (AUC, μg × hr/ml) and maximum drug concentration (Cmax, μg/ml).

Table 5

| | Cmax (μg/mL) | AUC (mg x hr/mL) |
|---|---|---|
| Type-A crystal | 3.6 | 5.1 |
| Type-C crystal | 2.0 | 3.5 |

As is apparent from the above result, type-A crystal has higher concentration in plasma than type-C crystal.

[Formulation example]

Formulation example 1:

The following components were admixed in a conventional method and punched out to give 100 tablets each containing 50 mg of active ingredient.

| | |
|---|---|
| · Type-A crystal of N-hydroxy-2(S)-methyl-5-ethoxymethoxy-4(S)-[N-(4- phenoxyohenylcarbonyl)amino] pentanamide | 5.0 g |
| · Carboxymethylcellulose calcium (disintegrating agent) | 0.2 g |
| · magnesium stearate (lubricating agent) | 0.1 g |
| · microcrystalline cellulose | 4.7 g |

Formulation example 2

The following components were admixed in a conventional method and punched out to give 100 tablets each containing 50 mg of active ingredient.

| | |
|---|---|
| ·Type-B crystal of N-hydroxy-2(S)-methyl-5-ethoxymethoxy-4(S)-[N-(4-phenoxyohenylcarbonyl)amino] pentanamide | 5.0 g |
| ·Carboxymethylcellulose calcium (disintegrating agent) | 0.2 g |
| ·Magnesium stearate (lubricating agent) | 0.1 g |
| ·microcrystalline cellulose | 4.7 g |

## Claims

1. A type-A crystal of N-hydroxy-2(S)-methyl-5-ethoxymethoxy-4(S)-[N-(4-phenoxyphenylcarbonyl)amino]pentanamide **characterized by** diffraction angle (2 θ ), half bandwidth and relative intensity represented by table 1 in X-ray powder diffraction spectrum obtained by Cu-Kα ray.

Table 1

| diffraction angle (2θ) | half bandwidth | relative intensity |
|---|---|---|
| 5.4 | 0.3 | medium |
| 6.7 | 0.2 | medium |
| 12.5 | 0.3 | medium |
| 17.0 | 0.3 | medium |
| 17.9 | 0.3 | strong |
| 21.4 | 0.3 | medium |
| 21.9 | 0.3 | strong |
| 23.4 | 0.3 | rather strong |
| 26.6 | 0.3 | medium |

2. A type-B crystal of N-hydroxy-2(S)-methyl-5-ethoxymethoxy-4(S)-[N-(4-phenoxyphenylcarbonyl)amino]pentanamide **characterized by** diffraction angle (2 θ ), half bandwidth and relative intensity represented by table 2 in X-ray powder diffraction spectrum obtained by Cu-Kα ray.

Table 2

| diffraction angle (2θ) | half bandwidth | relative intensity |
|---|---|---|
| 6.5 | 0.1 | strong |
| 8.4 | 0.1 | medium |

Table 2 (continued)

| diffraction angle (2θ) | half bandwidth | relative intensity |
|---|---|---|
| 11.3 | 0.1 | medium |
| 14.5 | 0.1 | medium |
| 16.8 | 0.2 | medium |
| 19.3 | 0.1 | rather strong |
| 20.2 | 0.1 | medium |
| 22.1 | 0.2 | medium |
| 25.0 | 0.1 | medium |

**3.** A method for the preparation of type-A crystal described in claim 1, **characterized by** recrystallization of crude compound of N-hydroxy-2(S)-methyl-5-ethoxymethoxy-4(S)-[N-(4-phenoxyphenylcarbonyl)amino]pentanamide from a mixture of alcohol solvent and water.

**4.** A method for the preparation of type-B crystal described in claim 1, **characterized by** recrystallization of crude compound of N-hydroxy-2(S)-methyl-5-ethoxymethoxy-4(S)-[N-(4-phenoxyphenylcarbonyl)amino]pentanamide from a mixture of tetrahydrofuran and cyclohexane.

**5.** A pharmaceutical composition comprising type-A crystal described in claim 1 or type-B crystal described in claim 2.

**6.** A solid pharmaceutical composition comprising type-A crystal described in claim 1 or type-B crystal described in claim 2.

**7.** A matrix metalloproteinase inhibitor comprising type-A crystal described in claim 1 or type-B crystal described in claim 2 as active ingredient.

**8.** A pharmaceutical agent for the treatment and/or prophylaxis of rheumatoid arthritis, arthrosteitis, pathological bone resorption, osteoporosis, periodontitis, interstitial nephritis, arteriosclerosis, pulmonary emphysema, cirrhosis hepatis, corneal damages, diseases of metastasis and infiltration of cancer cells and proliferation, autoimmune diseases, diseases caused by vascular emigration or infiltration of leukocytes, angiogenesis, multiple sclerosis, aortic aneurysm, endometriosis, osteoarthritis, diseases caused by constrictive vessel lesion, comprising type-A crystal described in claim 1 or type-B crystal described in claim 2 as active ingredient.

FIG. 1

X-RAY POWDER DIFFRACTION SPECTRUM OF TYPE-A CRYSTAL

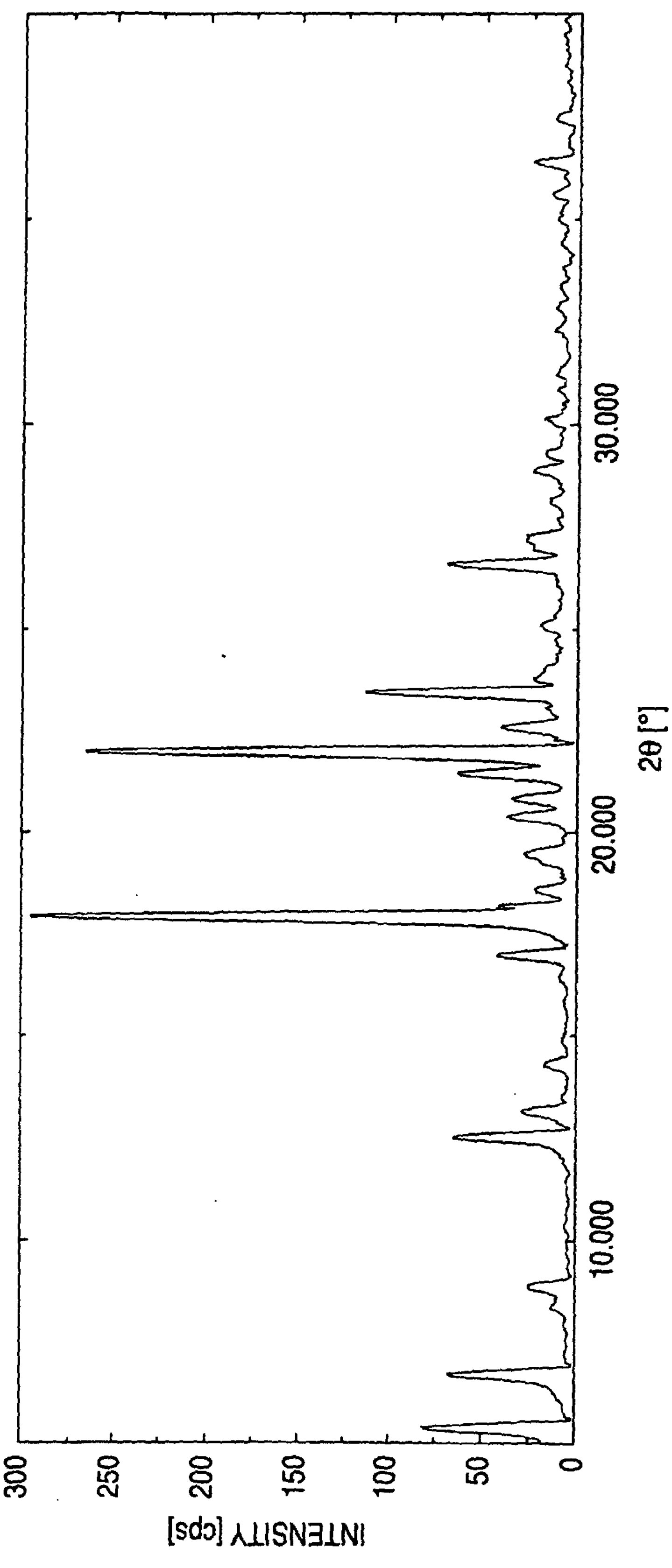

FIG. 2

DSC CHART OF TYPE-A CRYSTAL

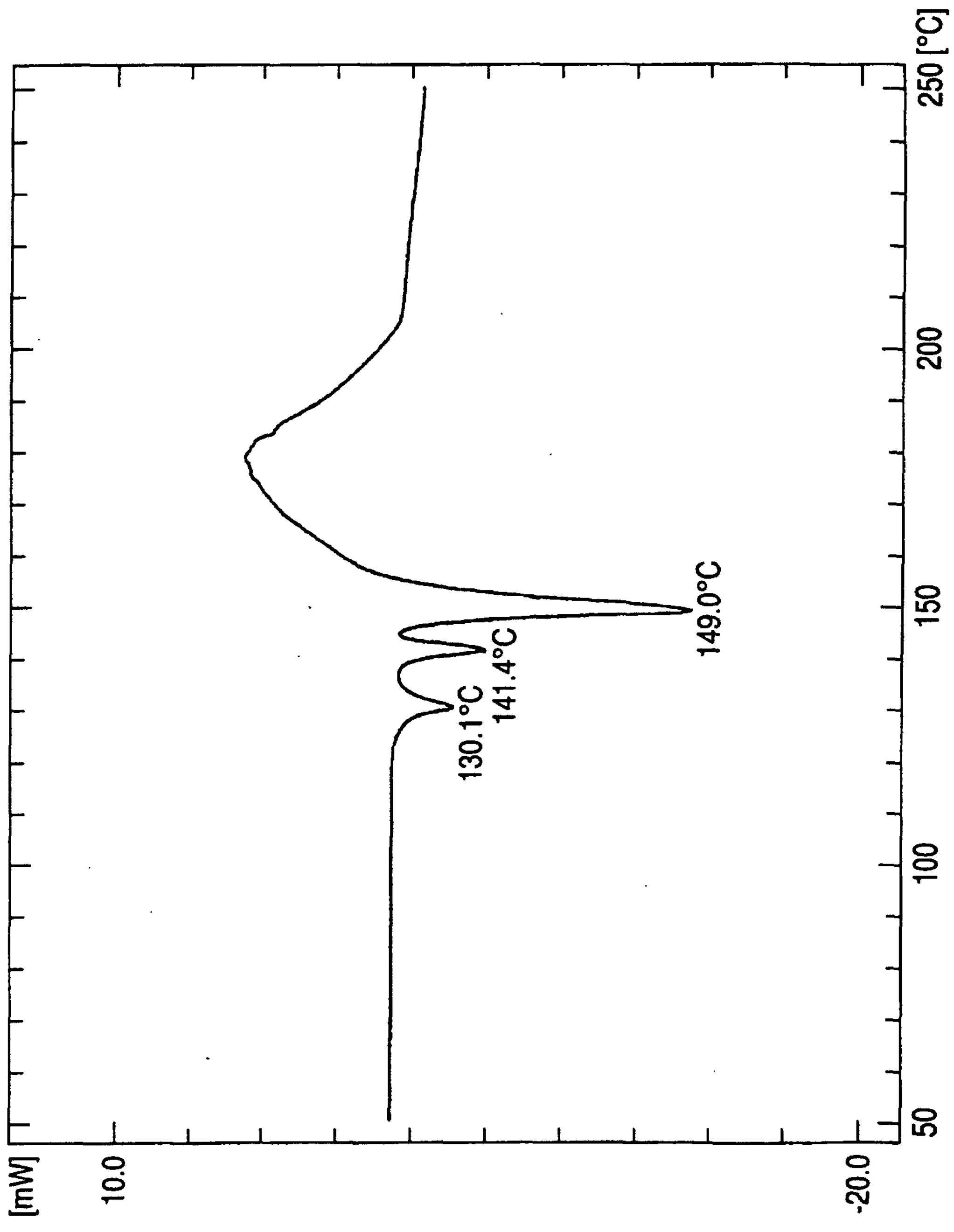

FIG. 3

IR SPECTRUM OF TYPE-A CRYSTAL

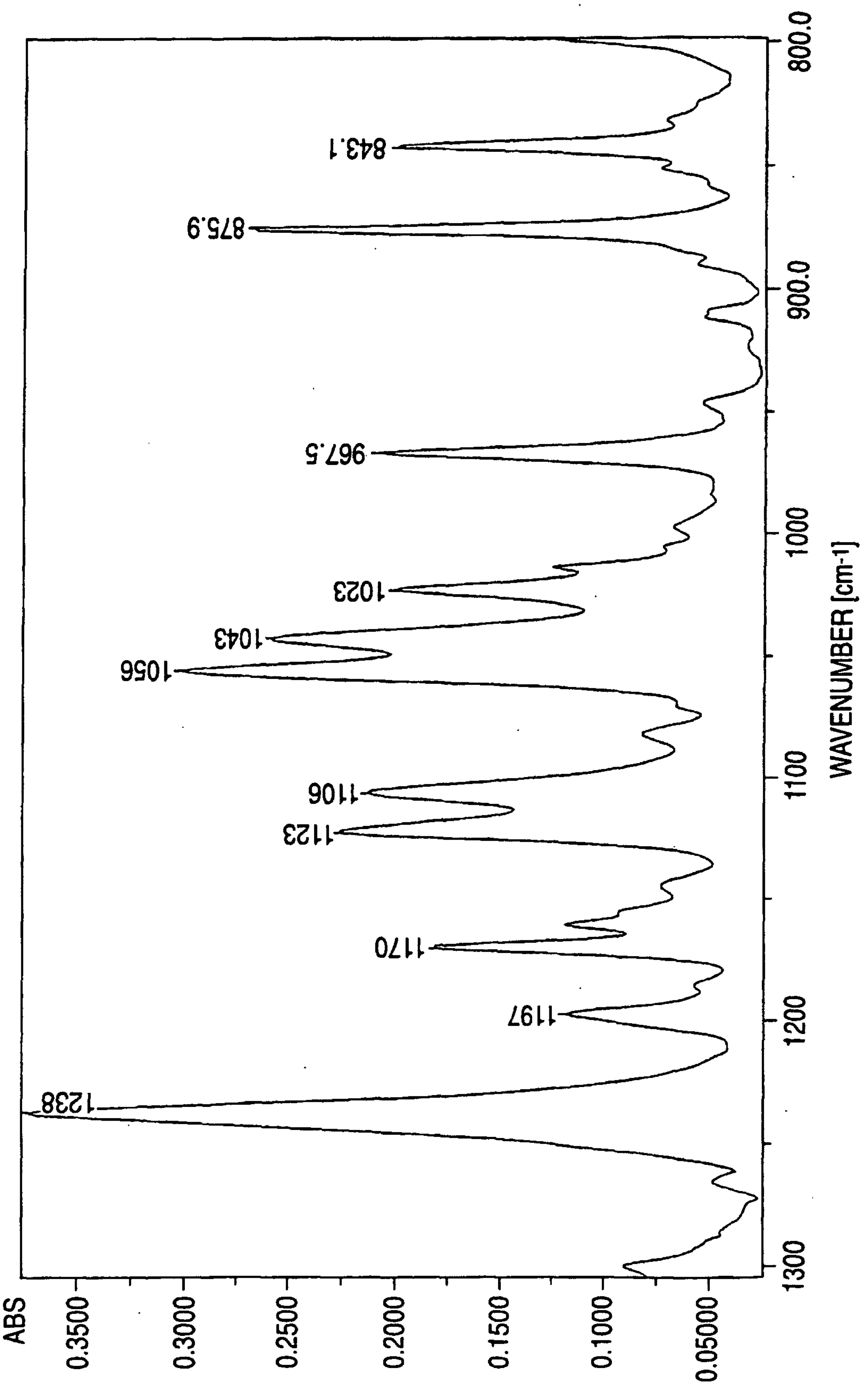

FIG. 4

X-RAY POWDER DIFFRACTION SPECTRUM OF TYPE-B CRYSTAL

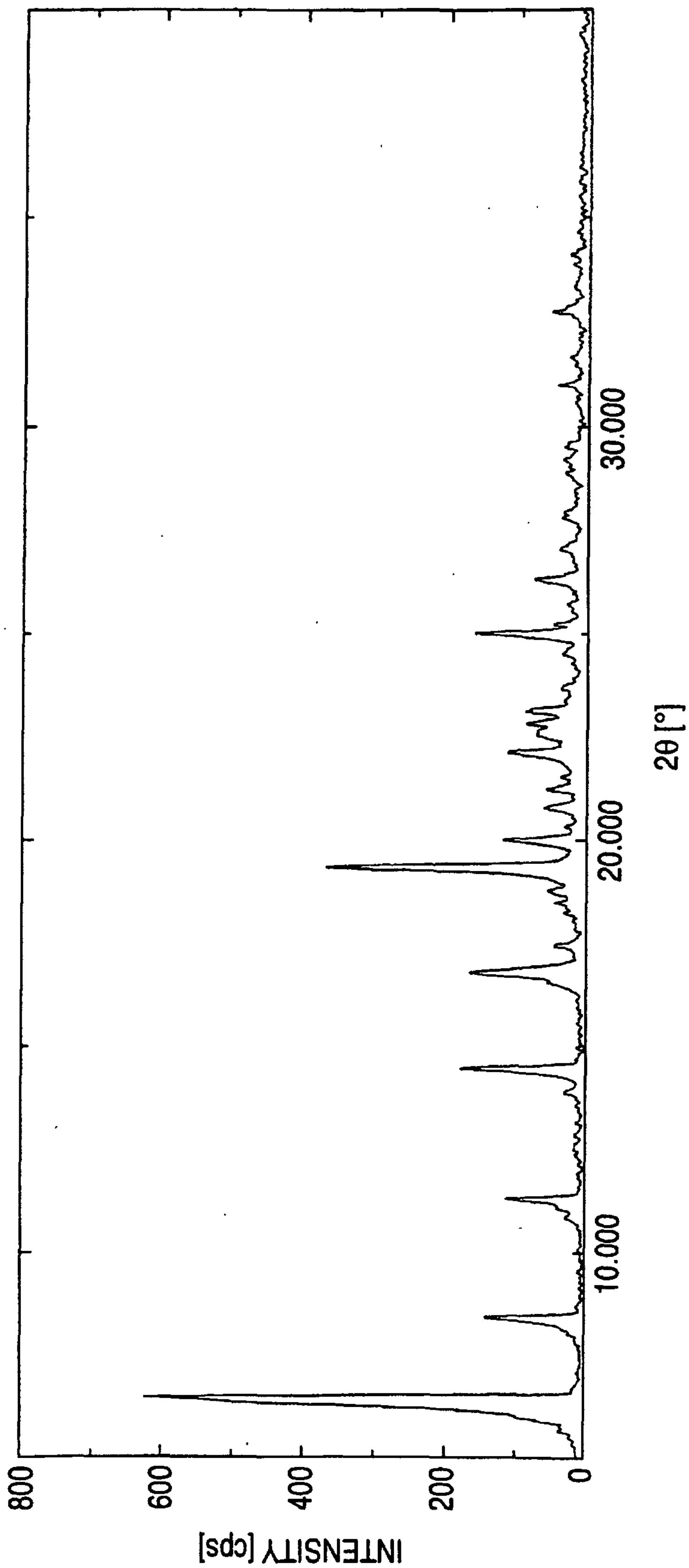

FIG. 5

DSC CHART OF TYPE-B CRYSTAL

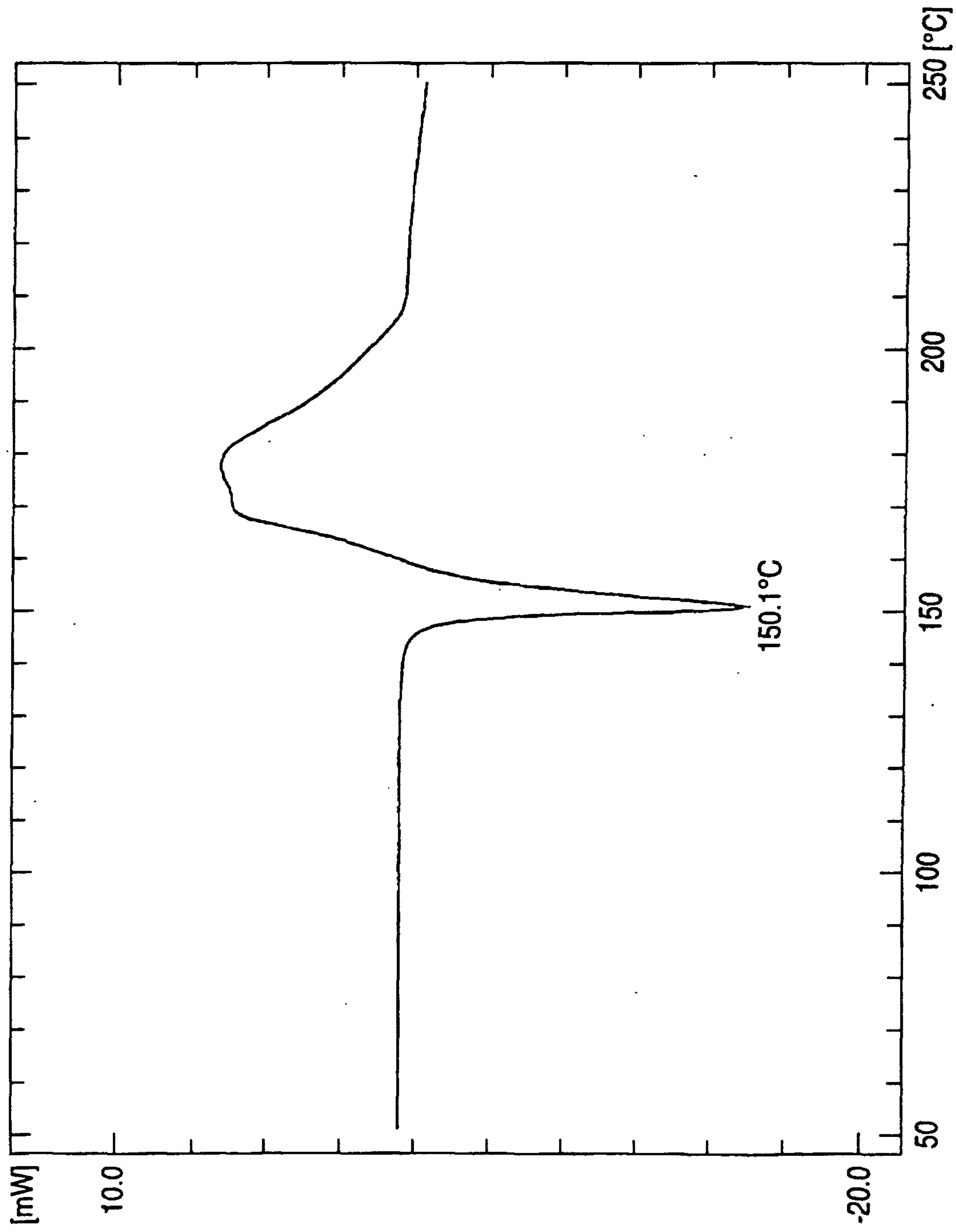

FIG. 6

IR SPECTRUM OF TYPE-B CRYSTAL

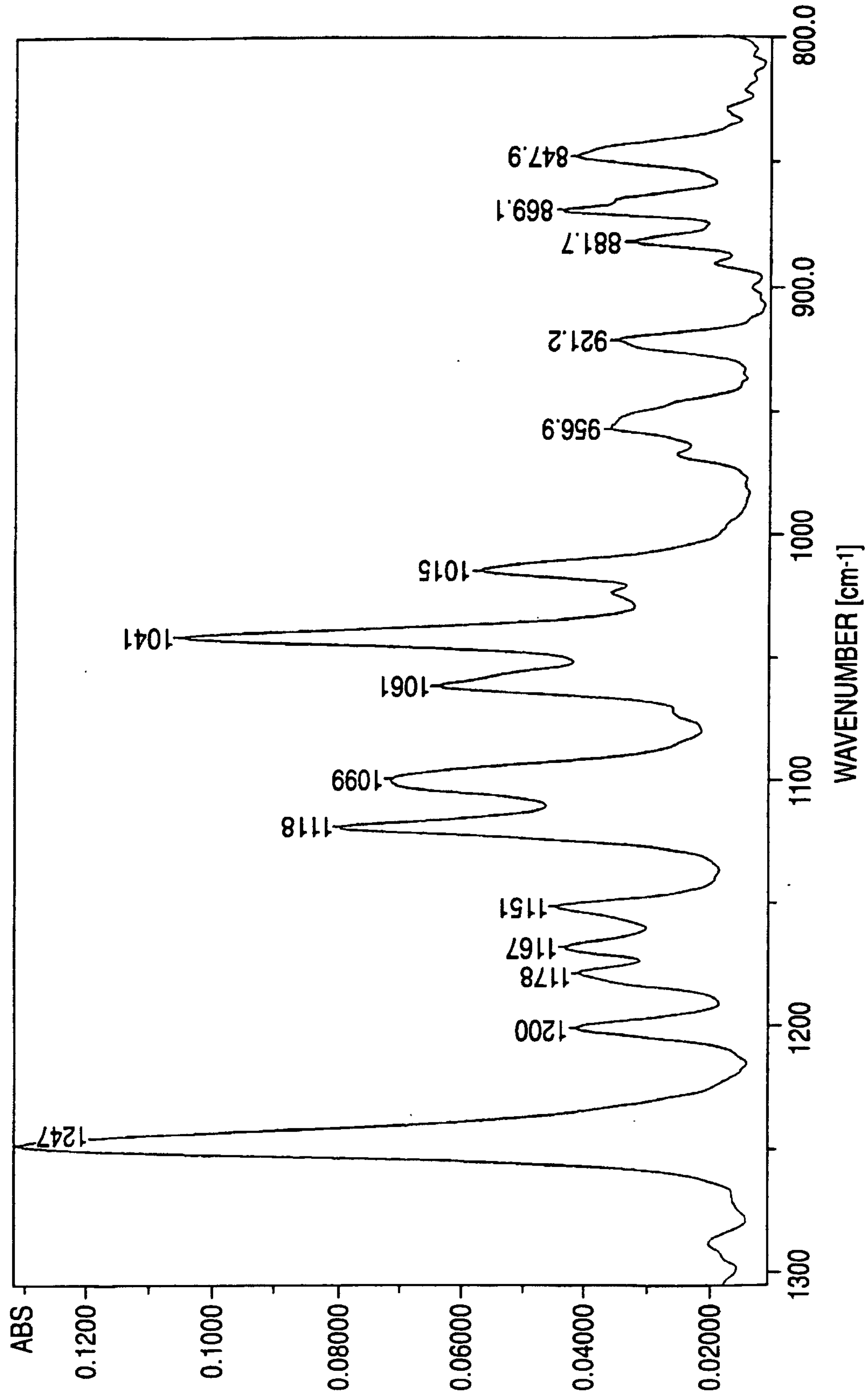

FIG. 7

X-RAY POWDER DIFFRACTION SPECTRUM OF TYPE-C CRYSTAL

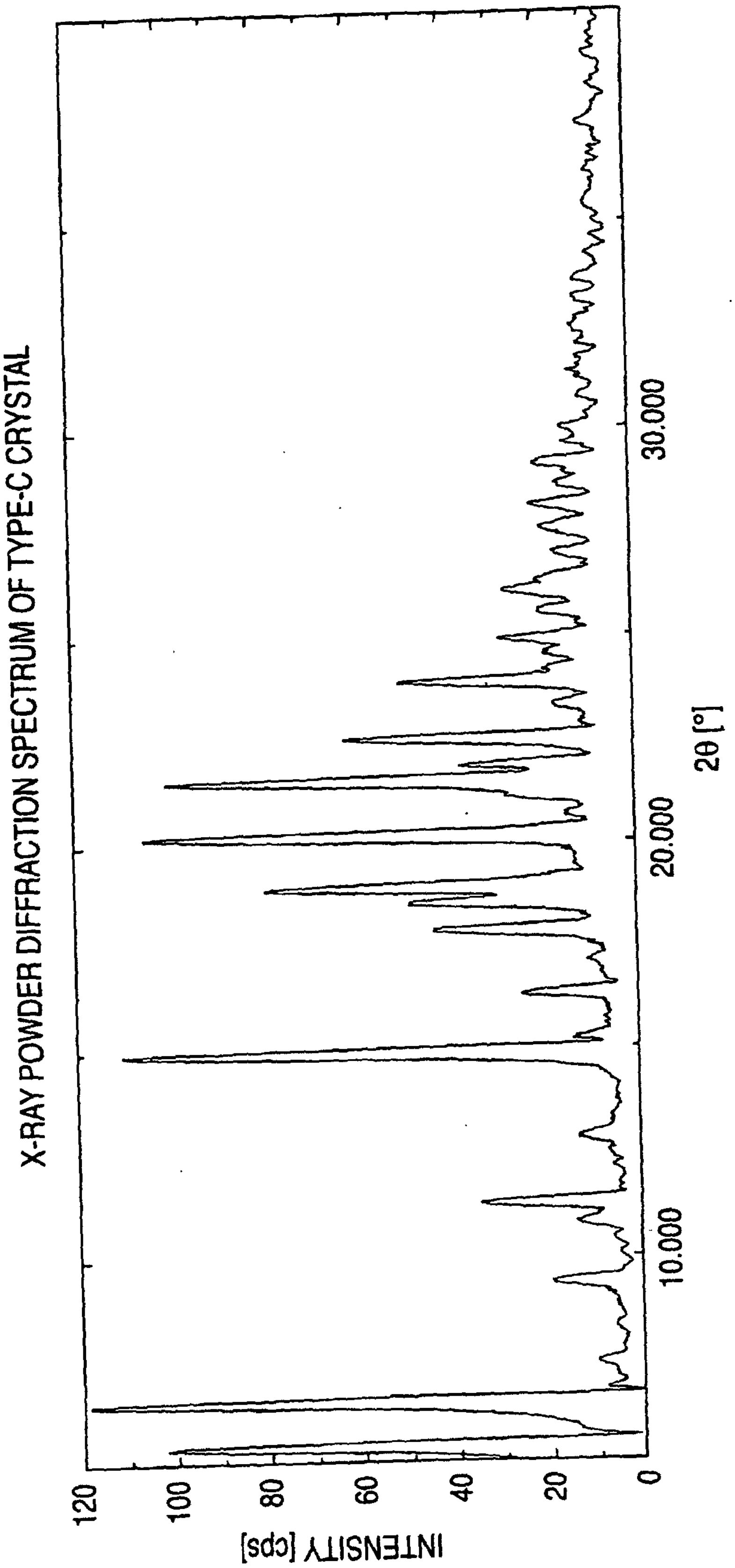

FIG. 8

DSC CHART OF TYPE-C CRYSTAL

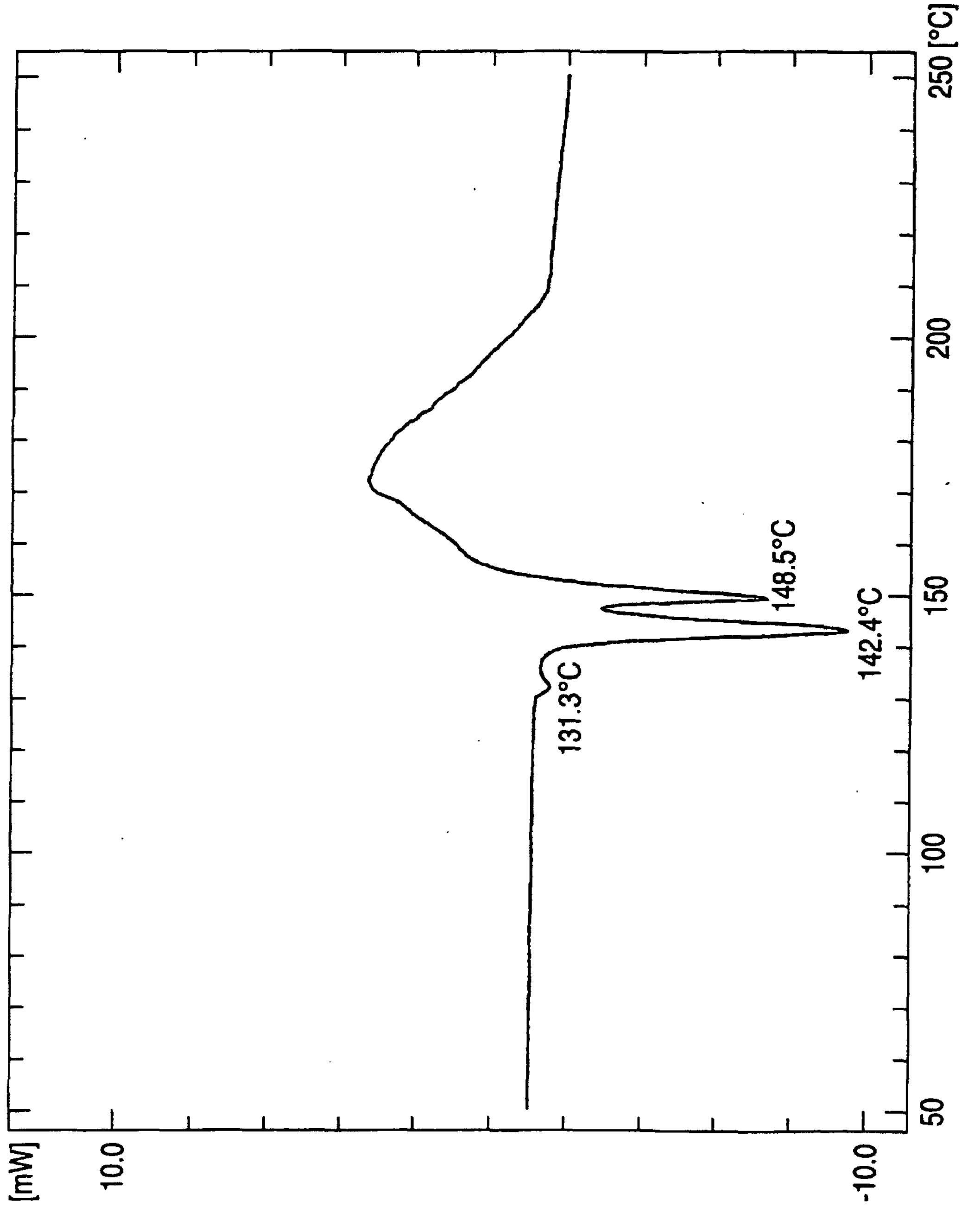

FIG. 9

IR SPECTRUM OF TYPE-C CRYSTAL

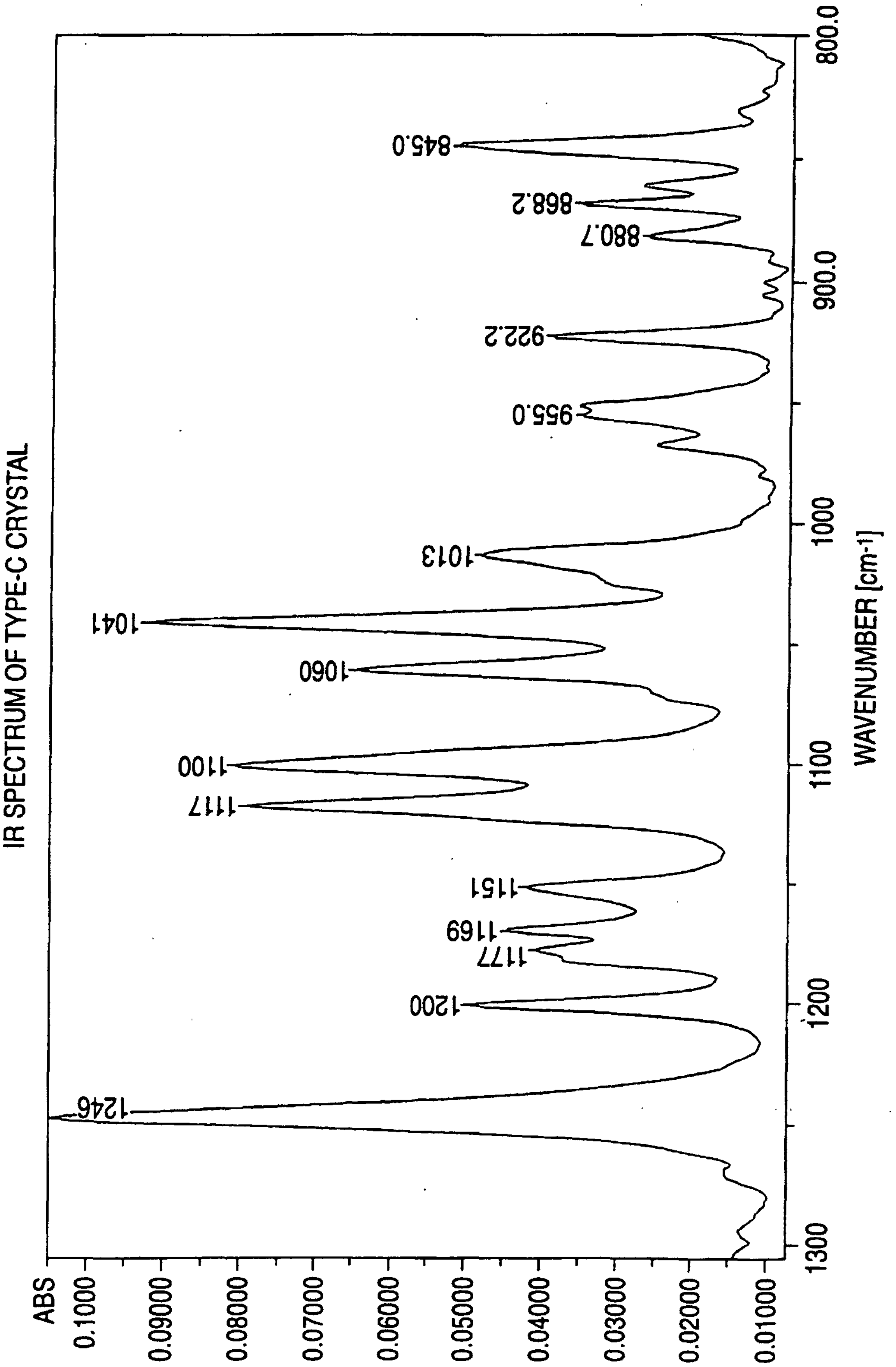

# FIG. 10

MULTIPLE CHART OF IR SPECTRA OF THE CRYSTALS PREPARED IN REFERENCE EXAMPLE 2 AND COMPARATIVE EXAMPLE 1

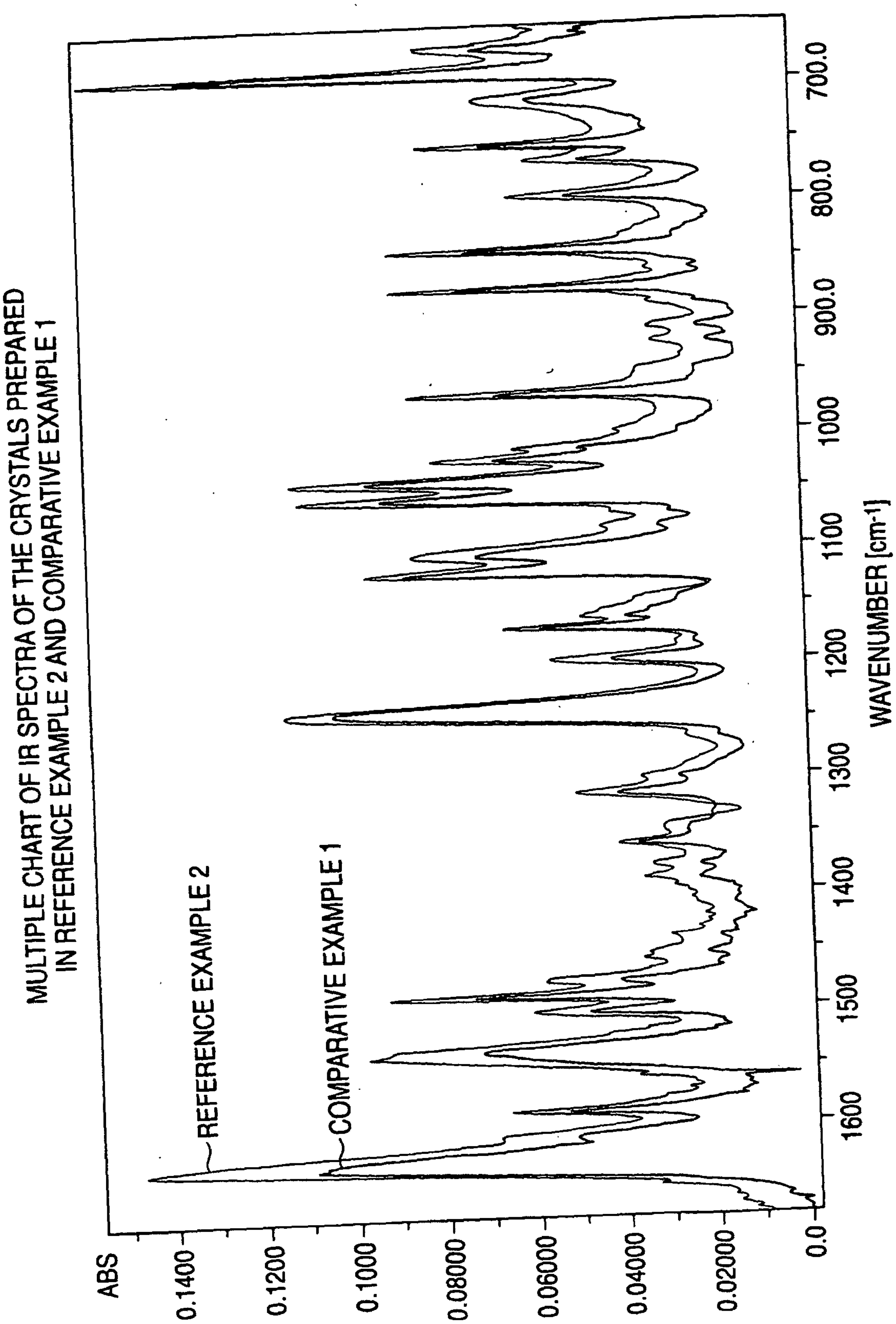

FIG. 11

MULTIPLE CHART OF X-RAY POWDER DIFFRACTION SPECTRA OF TYPE-A, B AND C CRYSTALS

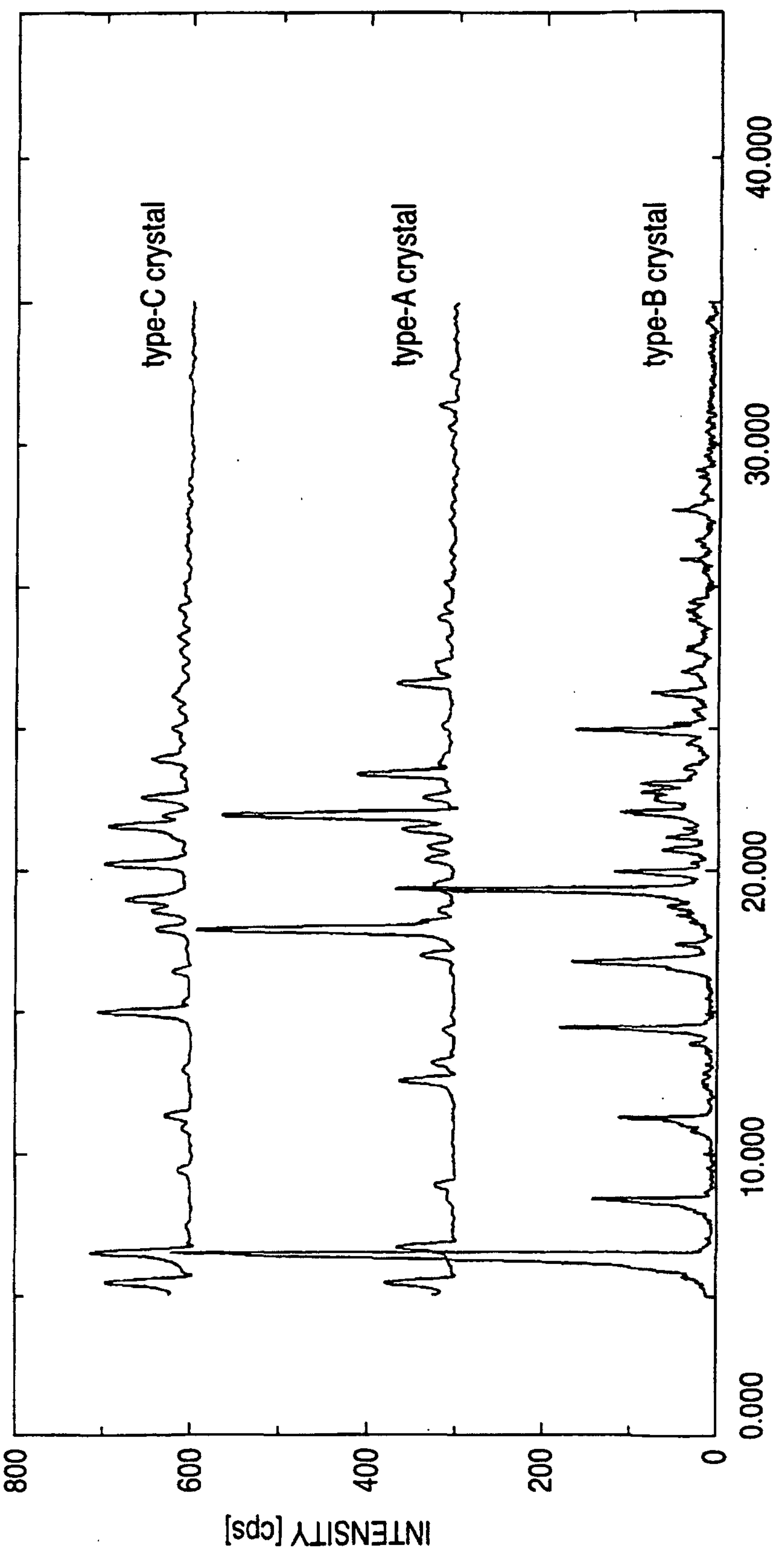

FIG. 12

MULTIPLE CHART OF DSC OF TYPE-A, B AND C CRYSTALS

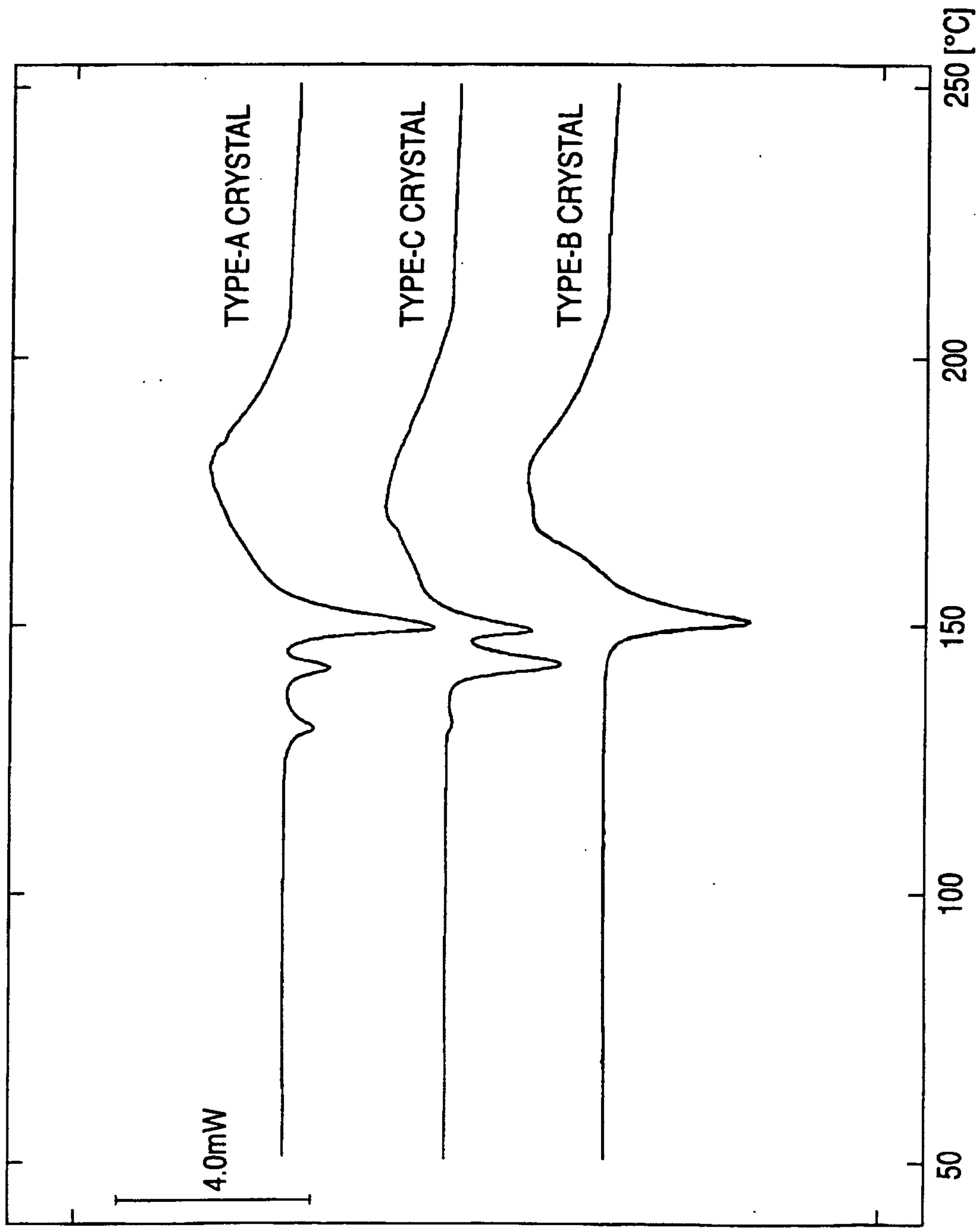

FIG. 13

MULTIPLE CHART OF IR SPECTRA OF TYPE-A, B AND C CRYSTALS

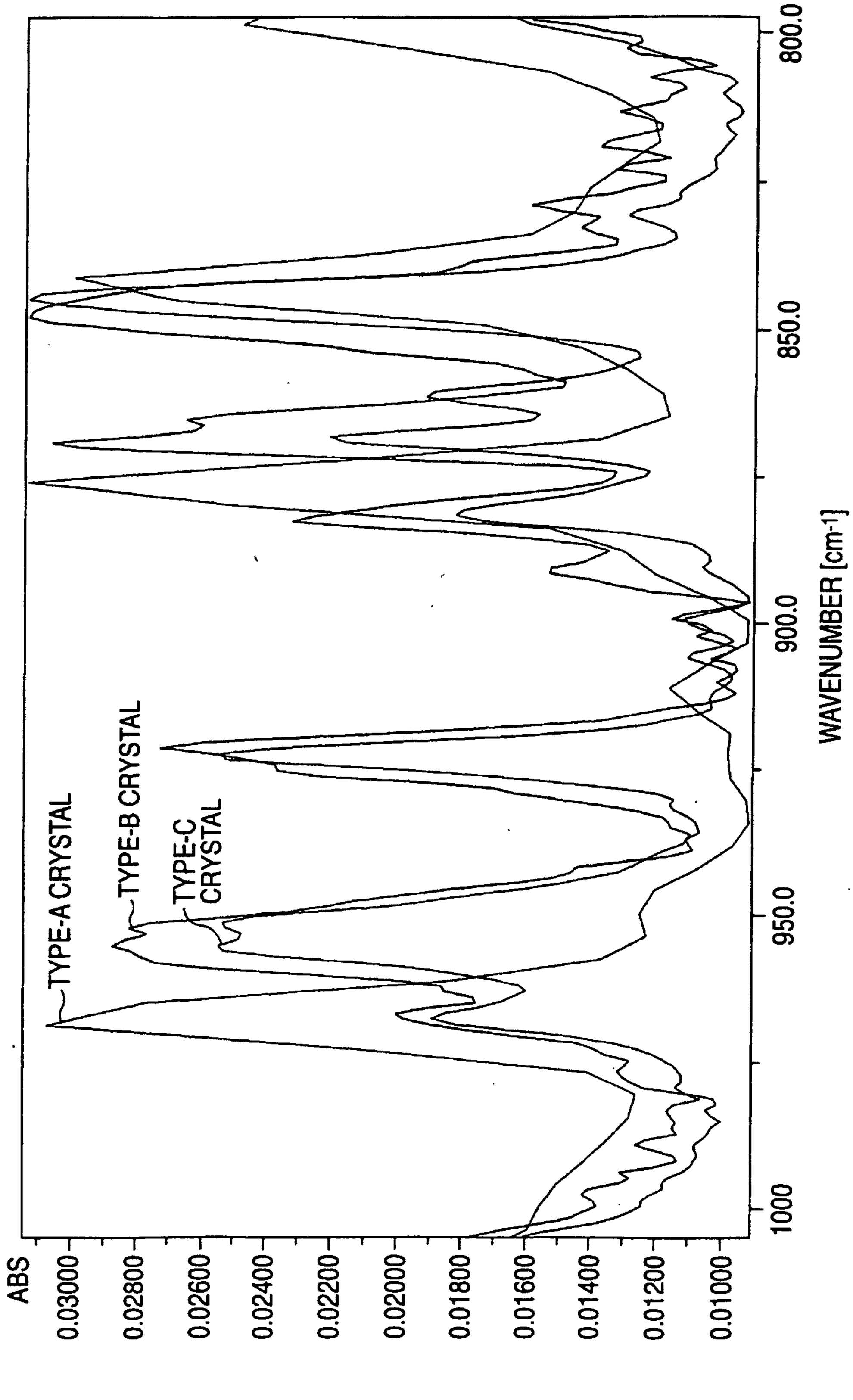

FIG. 14

DSC CHART OF INITIAL TYPE-A CRYSTAL

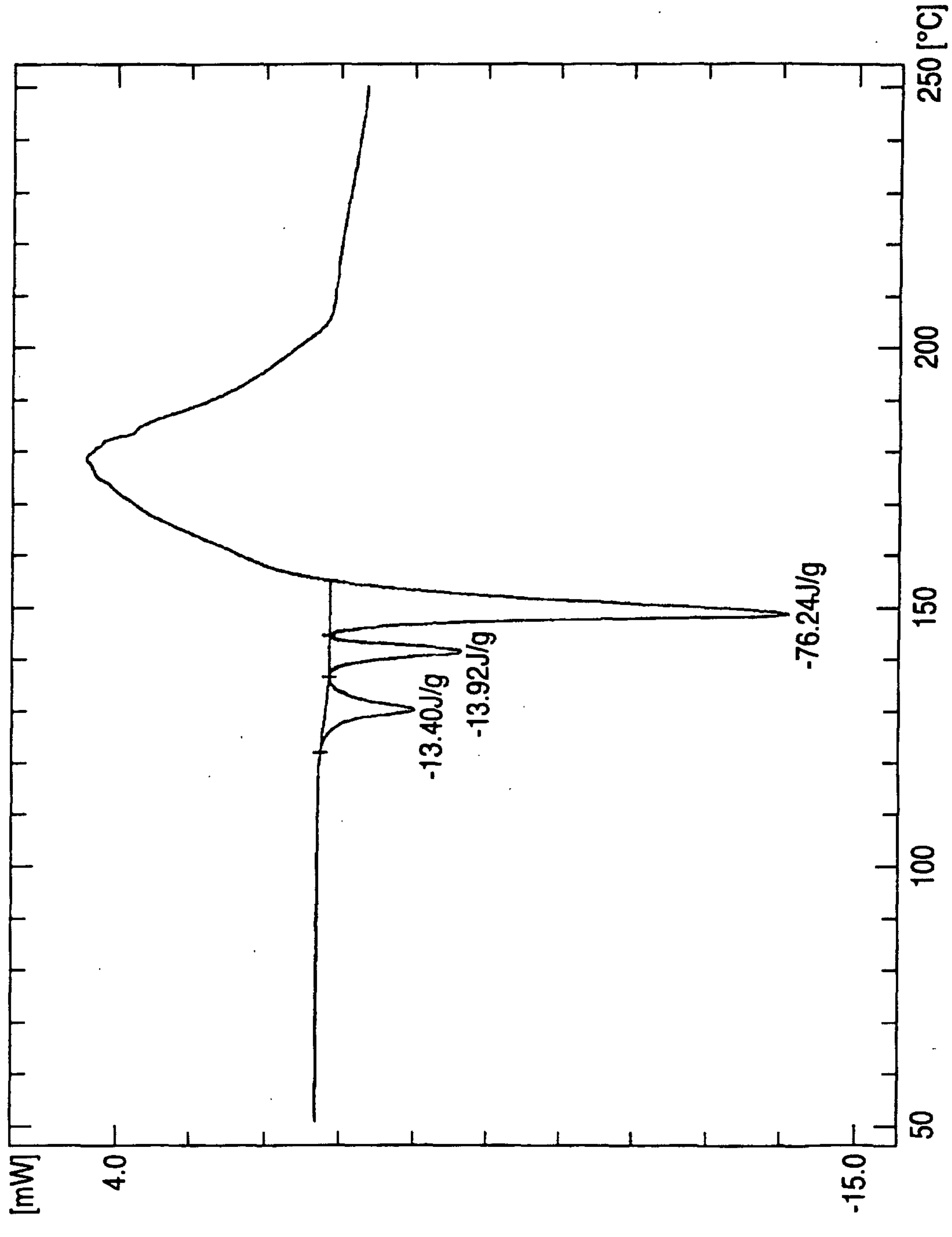

FIG. 15

DSC CHART OF TYPE-A CRYSTAL, PRESERVED FOR 1 MONTH UNDER THE CONDITION OF 40°C, 75%RH

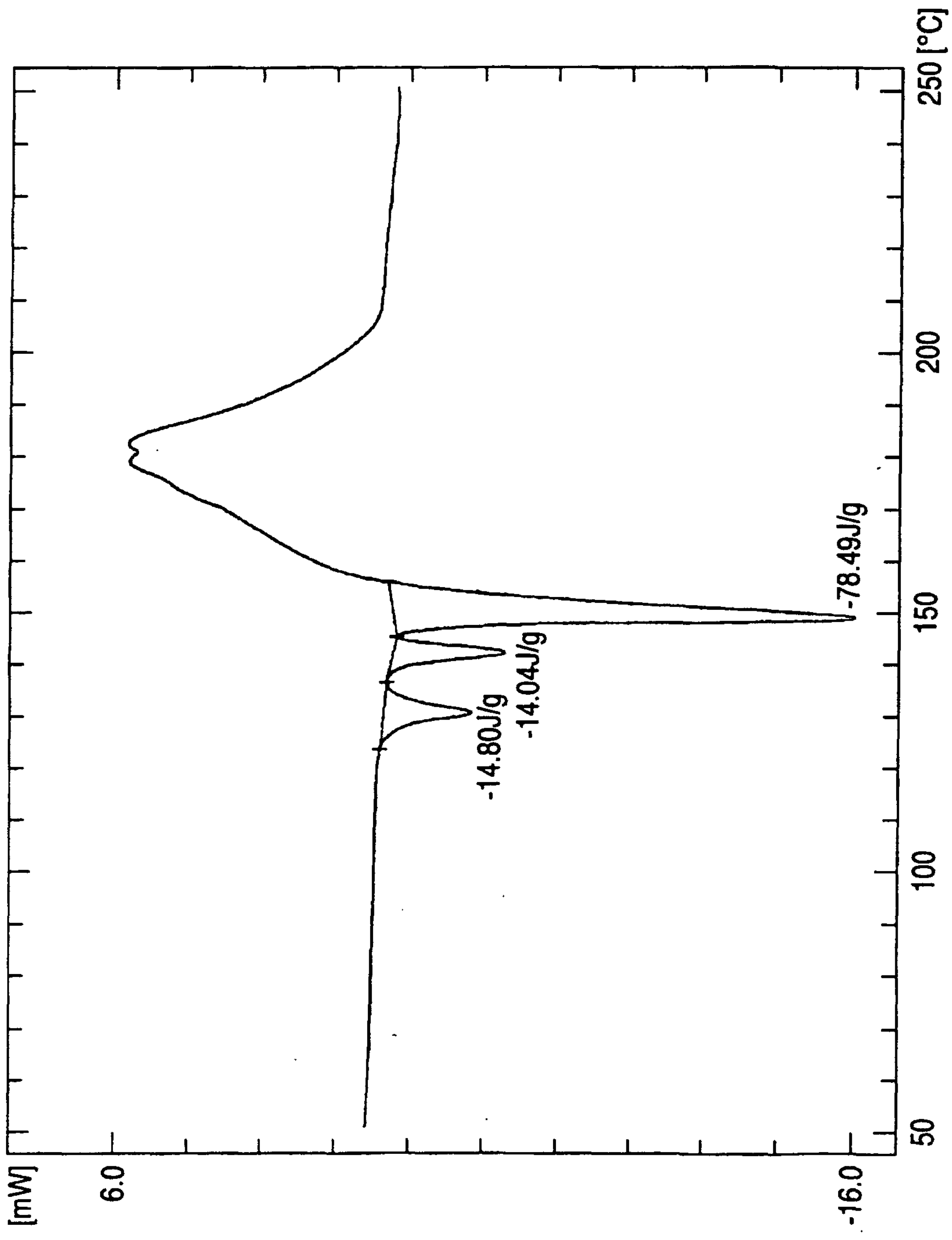

FIG. 16

DSC CHART OF TYPE-A CRYSTAL, PRESERVED FOR 6 MONTH AT 40°C

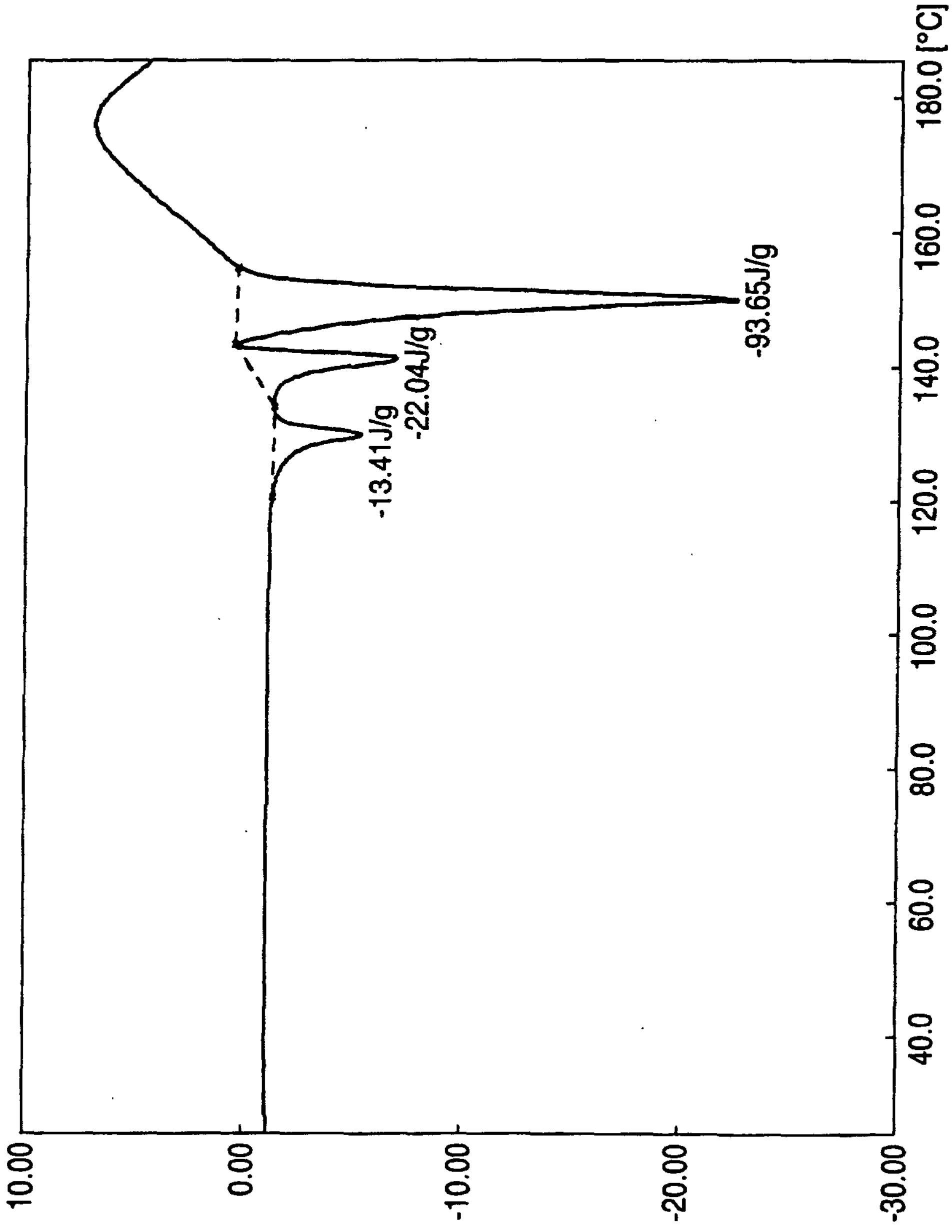

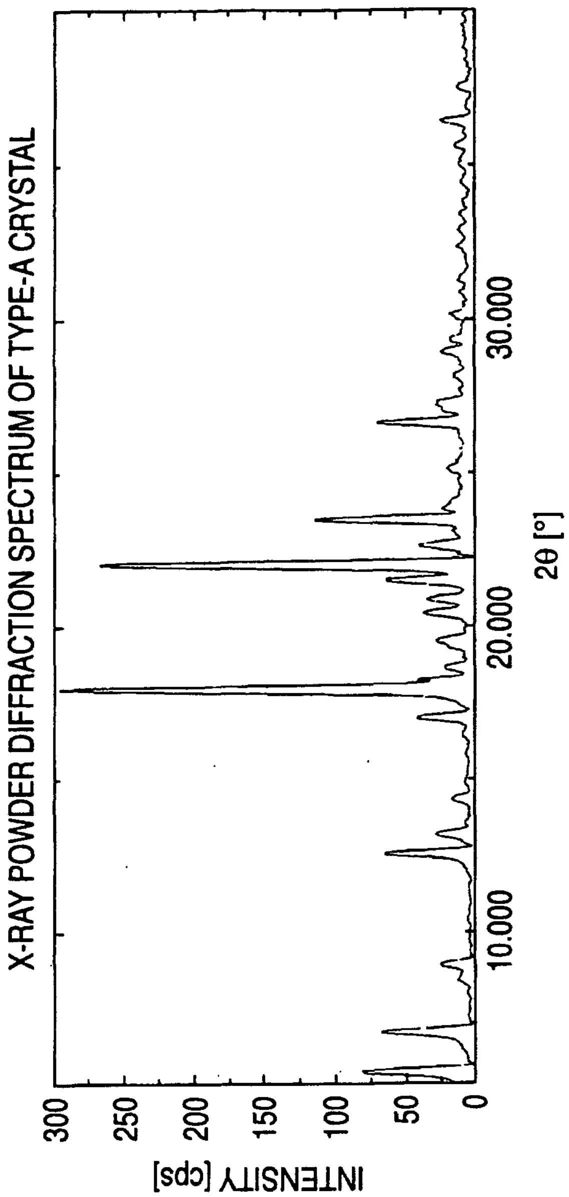


FIG. 17A

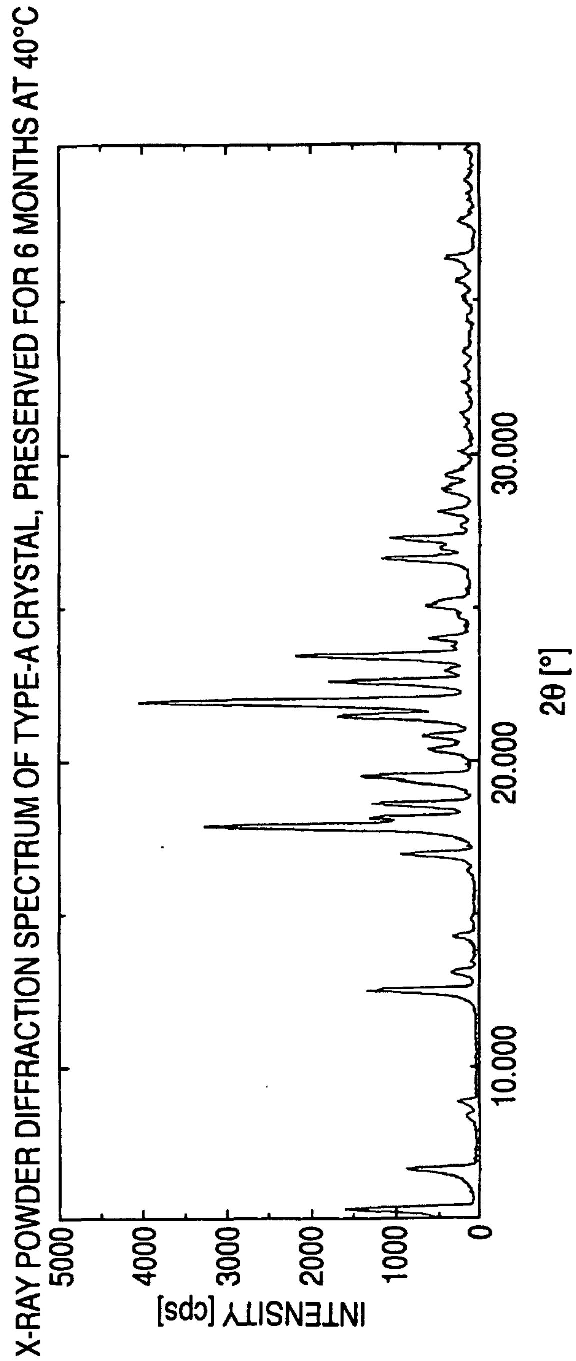


FIG. 17B

FIG. 18

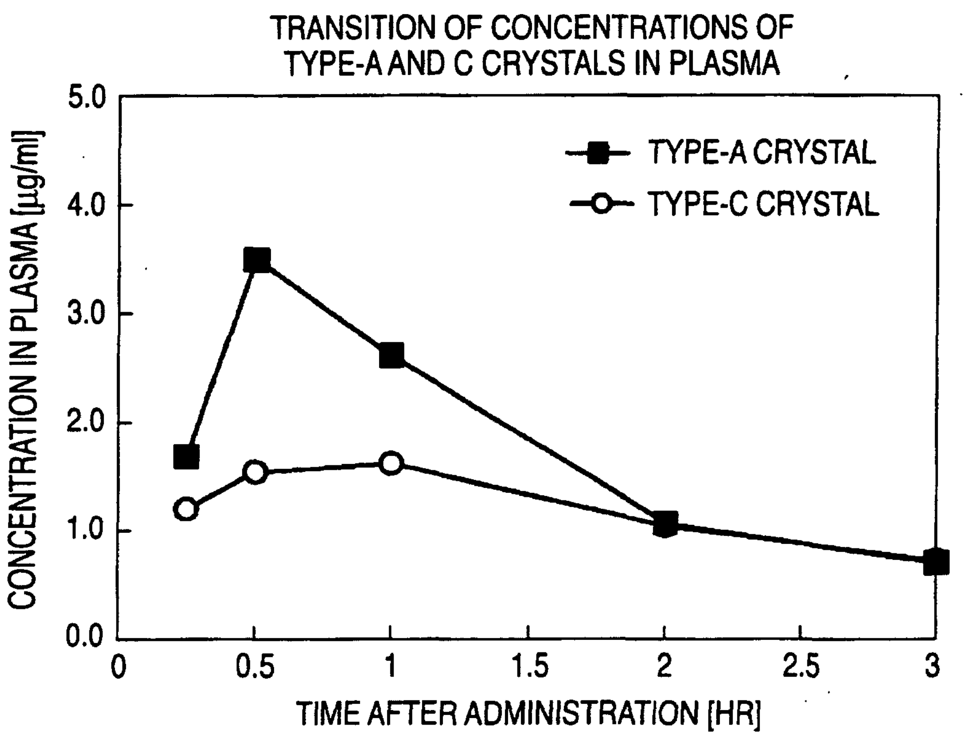
TRANSITION OF CONCENTRATIONS OF
TYPE-A AND C CRYSTALS IN PLASMA
CONCENTRATION IN PLASMA [μg/ml]
5.0
4.0
3.0
2.0
1.0
0.0
TYPE-A CRYSTAL
TYPE-C CRYSTAL
0
0.5
1
1.5
2
2.5
3
TIME AFTER ADMINISTRATION [HR]

## INTERNATIONAL SEARCH REPORT

International application No.
PCT/JP01/07775

A. CLASSIFICATION OF SUBJECT MATTER
Int.Cl[7] C07C259/06, A61K31/166, A61P43/00, 29/00, 19/02, 19/10, 1/02, 13/12, 9/10, 11/00, 1/16, 27/02, 35/00, 35/04, A61P37/06, 9/00, 15/00

According to International Patent Classification (IPC) or to both national classification and IPC

B. FIELDS SEARCHED

Minimum documentation searched (classification system followed by classification symbols)
Int.Cl[7] C07C259/00, A61K31/00

Documentation searched other than minimum documentation to the extent that such documents are included in the fields searched

Electronic data base consulted during the international search (name of data base and, where practicable, search terms used)
REGISTRY(STN), CA(STN)

C. DOCUMENTS CONSIDERED TO BE RELEVANT

| Category* | Citation of document, with indication, where appropriate, of the relevant passages | Relevant to claim No. |
|---|---|---|
| A | EP 1024134 A1 (Ono Pharmaceutical Co.), 02 August, 2000 (02.08.00), & JP 2001-172245 A | 1-8 |
| A | A. YAMADA et al., "ONO-4817, an orally active matrix metalloproteinase inhibitor, prevents lipopolysaccharide- induced proteoglycan release from the joint cartilage in guinea pigs", Inflammation Res., 2000, Vol. 49, No. 4, pages 144-146 | 1-8 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 December, 2001 (03.12.01) | 18 December, 2001 (18.12.01) |
| Name and mailing address of the ISA/ Japanese Patent Office | Authorized officer |
| Facsimile No. | Telephone No. |

Form PCT/ISA/210 (second sheet) (July 1992)